(19) Europäisches Patentamt
European Patent Office
Office européen des brevets

(11) Veröffentlichungsnummer: **0 374 866 B1**

(12) **EUROPÄISCHE PATENTSCHRIFT**

(45) Veröffentlichungstag der Patentschrift: **15.06.94**

(51) Int. Cl.[5]: **C07C 233/05**, C08F 20/60, C08F 20/36, B05D 1/20

(21) Anmeldenummer: **89123522.8**

(22) Anmeldetag: **20.12.89**

Die Akte enthält technische Angaben, die nach dem Eingang der Anmeldung eingereicht wurden und die nicht in dieser Patentschrift enthalten sind.

(54) **Amphiphile Monomere mit gemischtkettiger Struktur und Polymere und Film aus mindestens einer monomolekularen Schicht daraus.**

(30) Priorität: **22.12.88 DE 3843194**

(43) Veröffentlichungstag der Anmeldung:
**27.06.90 Patentblatt 90/26**

(45) Bekanntmachung des Hinweises auf die Patenterteilung:
**15.06.94 Patentblatt 94/24**

(84) Benannte Vertragsstaaten:
**AT BE CH DE ES FR GB IT LI NL SE**

(56) Entgegenhaltungen:
**EP-A- 0 084 227**
**EP-A- 0 308 801**
**WO-A-83/03165**
**US-A- 2 945 878**
**US-A- 4 247 673**

**JOURNAL OF THE AMERICAN CHEMICAL SOCIETY, Band 109, 4. Februar 1987, Seiten788-798; H. RINGSDORF et al.: "Self-organization of polymeric lipids withhydrophilic spacers in side groups and main chain: Investigation in monolayersand multilayers"**

**JOURNAL OF THE AMERICAN CHEMICAL SOCIETY, Band 107, 10. Juli 1985, Seiten 4134-4141; H. RINGSDORF et al.: "Hydrophilic spacer groups in polymerizable lipids:Formation of biomembrane models from bulk polymerized lipids"**

(73) Patentinhaber: **HOECHST AKTIENGESELLSCHAFT**

**D-65926 Frankturt(DE)**

(72) Erfinder: **Lupo, Donald, Dr.**
**Am Holderbusch 28**
**D-6239 Eppstein/Tanus(DE)**
Erfinder: **Prass, Werner, Dr.**
**Bahnstrasse 19**
**D-6500 Mainz(DE)**
Erfinder: **Scheunemann, Ude, Dr.**
**Feldbergstrasse 12**
**D-6237 Liederbach(DE)**

**EP 0 374 866 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf spezielle amphiphile Monomere mit Alkylseitenketten und deren Polymere, auf einen Film aus mindestens einer monomolekularen Schicht dieser Moleküle auf einem festen Schichtträger (= sogenannte Schichtelemente) und Verfahren zur Herstellung der Monomeren, der Polymeren und der Schichtelemente.

Zur Herstellung von geordneten Schichten organischer Polymerer mit langkettigen Seitengruppen wird überwiegend das Langmuir-Blodgett (LB)-Verfahren benutzt. Bei diesem Verfahren werden Moleküle auf einer Wasseroberfläche gespreitet und durch Verkleinerung der Fläche pro Molekül die langen Alkylseitengruppen parallel angeordnet. Bei konstantem Schub werden die Moleküle durch Ein- und Austauchen auf ein Substrat aufgezogen. Pro Tauchgang wird dabei eine monomolekulare Schicht unter Erhalt ihrer Ordnung übertragen.

Für den Aufbau von LB-Filmen verwendet man amphiphile Moleküle, d.h. Moleküle, die ein hydrophiles Ende (einen "Kopf") und eine hydrophobes Ende (einen "Schwanz") haben. Um eine höhere Stabilität der LB-Filme zu erreichen, wurden auch schon polymere LB-Filme hergestellt.

Hierzu wurden einmal ungesättigte Amphiphile nach der Herstellung des Films polymerisiert. Man hat aber auch schon unmittelbar organische Polymere mit langen Alkylseitenketten zur Schichtherstellung eingesetzt (WO83/03165 und R. Elbert, A. Laschewsky und H. Ringsdorf, J.Am.Chem.Soc. 107, 4134-4141 (1985)). Bei beiden Arten von polymeren Filmen treten jedoch mehr Fehlstellen als bei monomeren Filmen auf. Bei der Polymerisation in der Schicht kommt es in nahezu allen Fällen zu einer Kontraktion in der Schicht, mit der Ausbildung von Fehlstellen. Bei Filmen aus Copolymeren, wie von A. Laschewsky, H. Ringsdorf, G. Schmidt und J. Schneider in J.Am.Chem.Soc. 109, 788-796 (1987) beschrieben, können durch das statistisch eingebaute hydrophile Comonomere unterschiedlich große hydrophile Schleifen entstehen, die zu Ungleichmäßigkeiten in der Schichtdicke führen.

LB-Schichten aus Homopolymeren lassen sich im Allgemeinen nur dann aufbauen, wenn die Alkylseitenkette und die polymerisierbare Einheit durch ein hydrophiles, flexibles Segment, den "hydrophilen Spacer" getrennt werden (R. Elbert, A. Laschewsky und H. Ringsdorf, J.Am.Chem.Soc. 107, 4134-4141 (1985)). Diese zeigen jedoch, vor allem bei der Herstellung von dickeren Schichten (200- 300 Monolagen) immer wieder Störungen im Schichtaufbau, die wahrscheinlich durch eine immer noch zu hohe Viskosität und damit eine zu geringe Flexibilität der polymeren LB-Schicht verursacht werden.

Es bestand daher die Aufgabe, Monomere herzustellen, deren Homopolymere eine hohe Flexibilität zeigen und sich besonders gut, auch bei tieferen Temperaturen, auf Schichtträger übertragen lassen.

Die vorliegende Erfindung löst diese Aufgabe. Sie beruht auf der Beobachtung, daß bei Amphiphilen mit zwei unterschiedlich langen Alkylketten die Flexibilität in Monoschichten stark erhöht ist. Dies trifft auch für die Polymeren zu. Zusätzlich ist durch die unterschiedlich langen Alkylreste die Möglichkeit der "Interdigitation" von Alkylketten zweier benachbarter Monolagen im LB-Film gegeben. Dadurch wird die Haftung im hydrophoben Bereich des LB-Films erhöht.

Gegenstand der Erfindung sind amphiphile Monomere mit gemischtkettigen Strukturen der Formel

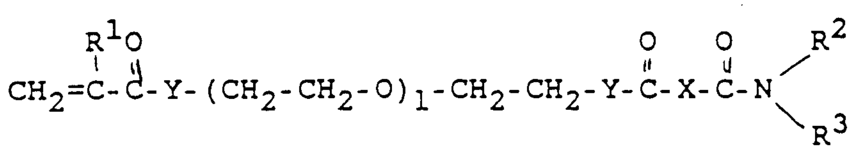

(I)

wobei

Y -O- oder -NH-

X eine Gruppe der Formel $-(CH_2)_n-$ oder $-(CH_2-O-CH_2)_n-$

l eine ganze Zahl von 0 bis 10,

n eine ganze Zahl von 1 bis 10,

$R^1$ Wasserstoff, Methyl, Chlor, Cyan, Fluor oder Brom,

$R^2$ $C_1$-$C_{24}$-Alkyl oder $C_1$-$C_{24}$-Fluoralkyl und

$R^3$ $C_8$-$C_{24}$-Alkyl oder $C_8$-$C_{24}$-Fluoralkyl bedeuten, mit der Maßgabe, daß die Gruppen $R^2$ und $R^3$ eine unterschiedliche Anzahl von C-Atomen enthalten.

Bei den Gruppen $R^2$ und $R^3$ sind die Alkylgruppen bevorzugt; diese können verzweigt oder unverzweigt sein. Die Fluoralkylgruppen können vollständig oder teilfluoriert sein, indem beispielsweise die Alkylgruppe mit einer Tri-fluormethyl-gruppe endet. In jedem Fall müssen die Gruppen $R^2$ und $R^3$ unterschiedlich lang

sein.

Diese Monomeren der Formel (1) können nach an sich bekannten Methoden auf folgendem Weg hergestellt werden:

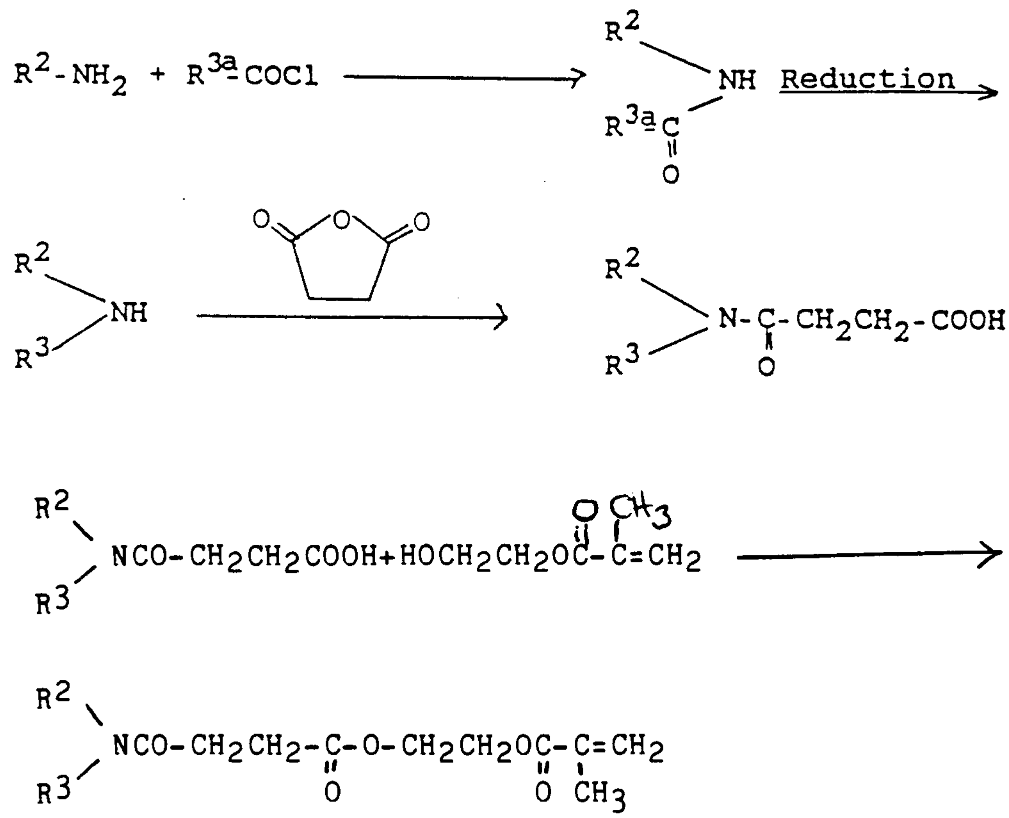


Dieses Verfahren ist in dem nachfolgenden Beispiel 1 näher erläutert.

Gegenstand der Erfindung sind außerdem Polymere, bevorzugt in Form von Filmen in mindestens einer monomolekularen Schicht, bestehend aus oder enthaltend ein Monomer der jeweiligen Formel I. Diese Polymere können vollständig aus den genannten Monomeren der Formel I aufgebaut sein, man kann diese neuen Monomeren aber auch mit anderen, hydrophoben oder hydrophilen Co-monomeren copolymerisieren. Derartige Copolymere enthalten bevorzugt 1 bis 20 Gew.-% des Monomeren der Formel I und 99 bis 80 Gew.-% eines hydrophoben Co-monomers bzw. 10 bis 99 Gew.-% des Monomeren der Formel I und 90 bis 1 Gew.-% eines hydrophilen Co-monomeren. Daneben können die Polymere auch aus verschiedenen Monomeren der Formel I aufgebaut sein. Die Herstellung dieser Homo- und Copolymere erfolgt durch radikalische Initiierung nach üblichen Verfahren. Als hydrophile Co-monomere sind bevorzugt wasserlösliche Vinylmonomere, wie beispielsweise Itaconsäure, Fumarsäure, Maleinsäure, Acryl-, Cyanoacryl- und Methacrylsäure oder deren wasserlösliche Derivate. Als Derivate sind insbesondere die Säureamide, Säurenitrile und der 2-Hydroxyethyl-Ester verwendbar.

Das hydrophobe Copolymere enthält neben einer polymerisierbaren Einheit, z.B. einer Vinyldoppelbindung, mindestens eine Alkylkette mit 10 bis 24 C-Atomen und/oder ist in Benzol löslich.

Die erfindungsgemäßen Polymere bzw. Filme können auch mit weiteren zweckdienlichen Komponenten abgemischt werden, beispielsweise mit Farbstoffen, amphiphilen Vernetzern, monomeren oder polymeren Amphiphilen.

Der Anteil dieser Zusatzstoffe in solchen Mischungen kann 10 bis 99 Gew.-% betragen.

Zur Herstellung von Filmen werden die erfindungsgemäßen Polymeren oder Mischungen, die diese Polymere enthalten, in einem im wesentlichen flüchtigen, mit Wasser nicht mischbaren Lösungsmittel gelöst und auf die Oberfläche einer wäßrigen Lösung in einer Filmwaage gebracht (=gespreitet). Aus der Abmessung der Oberfläche, dem Spreitvolumen und der Konzentration der Lösung wird die mittlere Fläche pro Repetiereinheit berechnet. Phasenübergänge bei der Kompression der Moleküle lassen sich in der Schub-Flächen Isotherme verfolgen.

Die Moleküle werden mit einer Barriere zusammengeschoben, wobei die Alkylketten bei wachsender Oberflächendichte im wesentlichen senkrecht zur Grenzschicht orientiert werden. Während der Kompression entsteht durch Selbstorganisation der Moleküle an der Grenzschicht ein hochgeordneter monomolekularer Film, dessen konstante Schichtdicke im wesentlichen durch die Kettenlänge der Alkylseitenketten der Polymeren und deren Tiltwinkel (das ist der Winkel, um den die Molekülketten auf der Wasseroberfläche gegen die Normale gekippt sind) bestimmt wird. Die typische Dicke eines solchen Films liegt bei 2 - 3 nm.

Der Film wird bei konstantem Schub durch Eintauchen oder Austauchen eines geeigneten Trägers unter Erhalt der Ordnung von der Wasseroberfläche abgenommen.

Als Subphase für die Monofilmherstellung dienen meist Wasser oder wäßrige Lösungen. Es sind aber auch andere Flüssigkeiten mit hoher Oberflächenspannung, wie z. B. Glyzerin, Glykol, Dimethylsulfoxid, Dimethylformamid oder Acetonitril verwendbar.

Als Träger kommen beliebige feste, vorzugsweise dimensionsstabile Substrate aus unterschiedlichen Materialien in Betracht. Die als Schichtträger dienenden Substrate können beispielsweise transparent oder lichtdurchlässig, elektrisch leitend oder isolierend sein. Das Substrat kann hydrophob oder hydrophil sein. Die Oberfläche des Substrats, auf die die LB-Schicht aufgebracht wird, kann hydrophobiert sein. Die zu beschichtende Oberfläche des Substrats sollte möglichst rein sein, damit die Ausbildung einer dünnen, geordneten Schicht nicht gestört wird. Insbesondere die Anwesenheit von oberflächenaktiven Stoffen auf der zu beschichtenden Oberfläche der Substrate kann die Schichtherstellung beeinträchtigen. Es ist möglich, die als Schichtträger dienenden Substrate auf der zu beschichtenden Oberfläche vor dem Aufbringen der LB-Filme zunächst mit einer Zwischenschicht zu versehen, um z.B. die Haftung des Films auf dem Substrat zu verbessern.

Als Materialien für die Substrate können beispielsweise Metalle, wie etwa Gold, Platin, Nickel, Palladium, Aluminium, Chrom, Niob, Tantal, Titan, Stahl und dergleichen verwendet werden. Andere geeignete Materialien für die Substrate sind Kunststoffe, wie beispielsweise Polyester, etwa Polyethylenterephthalat oder Polybutylenterephthalat, Polyvinylchlorid, Polyvinylidenchlorid, Polytetrafluorethylen, Polystyrol, Polyethylen oder Polypropylen.

Gleichermaßen kommen auch Halbleiter, wie Silizium, Germanium oder Galliumarsenid oder auch Glas, Siliziumdioxid, keramische Werkstoffe oder Celluloseprodukte für die Substrate in Betracht. Die Oberfläche von Glas und anderen hydrophilen Substraten kann, sofern erforderlich, in an sich bekannter Weise durch Umsetzung mit Alkylsilanen oder Hexamethyldisilazan hydrophobiert werden. Die Auswahl der Substratmaterialien richtet sich in erster Linie nach dem Verwendungszweck der aus dem erfindungsgemäßen Film hergestellten Schichtelemente. Für optische Elemente werden in der Regel transparente, lichtdurchlässige Substrate als Schichtträger eingesetzt. Werden die erfindungsgemäßen Schichtelemente beispielsweise in der Elektronik oder bei elektrochemischen Prozessen verwendet, dienen als Substrate insbesondere elektrisch leitfähige Materialien, wie Metalle oder metallische Oberflächenschichten, beispielsweise auf Kunststoffolien oder Glas.

Die als Träger für die erfindungsgemäßen Filme dienenden Substrate können, je nach Verwendungszweck, beliebige Formen aufweisen. Beispielsweise können sie film-, folien-, platten-, bandförmig oder auch zylinderförmig sein oder unter anderen beliebigen Formen aus gewählt werden. Im allgemeinen wird es sich bei den Schichtträgern um flache, ebene Substrate handeln, wie z.B. Filme, Folien, Platten, Bänder und dergleichen. Die zu beschichtende Oberfläche der Substrate ist vorzugsweise glatt, wie es für die Herstellung von LB-Filmen üblich ist. Bei flachen, ebenen Substraten können die erfindungsgemäßen Filme auf eine oder beide Oberflächen des Substrats aufgebracht werden.

Die erfindungsgemäßen Polymeren eignen sich sehr gut für die Herstellung einer Multischichtstruktur mit wenigen Defektstellen, wobei die Schichtherstellung bei für Polymere vergleichweise niedrigen Temperaturen möglich ist und die Schichtstruktur trotzdem auch bei höheren Temperaturen noch stabil ist.

Solche Filme auf Substraten eignen sich beispielsweise für optische Wellenleitersysteme oder zur Herstellung von Filtern für optische Zwecke. Aufgrund der geringen kritischen Oberflächenspannung sind die Filme auch zur Verbesserung der Reibungseigenschaften von Materialien, zur Herstellung von Schutzschichten sowie für weitere einschlägige Anwendungen geeignet.

Die Erfindung wird durch die nachfolgenden Beispiele näher erläutert.

Beispiel 1:

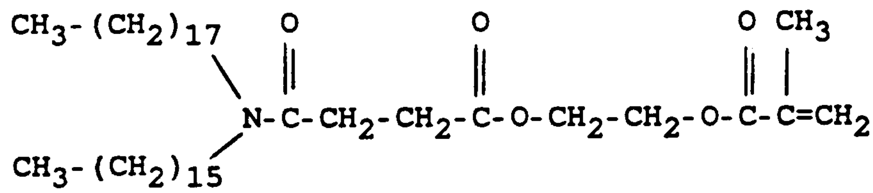

Zu einer Lösung von 26.95 g (0.1 mol) N-Octadecylamin und 12.14 g (0.12 mol) Triethylamin in 500 ml trockenem Methylenchlorid wird bei 0 °C innerhalb von 30 Minuten eine Lösung von 27.5 g (0.1 mol) Palmitinsäurechlorid in 100 ml trockenem Methylenchlorid zugetropft, 2 Stunden unter Kühlung bei 0 °C gerührt und unter Rühren auf Raumtemperatur erwärmt. Dann wird zu der entstandenen breiartigen Masse 500 ml wäßrige 1 M HCl-Lösung zugesetzt, bei Raumtemperatur 30 Minuten gerührt und das Produkt abgesaugt. Anschließend wird das Produkt aus Methylenchlorid umkristallisiert.
Es werden 20.8 g (82%) eines weißen Pulvers erhalten, das bei 96-97 °C schmilzt.
$^1$H-NMR ($CDCl_3$, 100 MHz)
$\delta$ = 0.9 (t,6H, $-CH_3$); 1.25 (m,58H, $-CH_2-$); 2.0-2.2 (t,2H, $-CH_2-CO$); 3.0-3.3 (m, 2H, $N-CH_2$); 5.2-5.5 (m,1H, -NH)
10 g (0.462 mol) $LiAlH_4$ werden in 100 ml trockenem Tetrahydrofuran suspendiert und bei Raumtemperatur innerhalb von 20 Minuten eine Suspension von 33 g (65 mmol) Palmitinsäureoctadecylamid in 500 ml trockenem Tetrahydrofuran zudosiert. Nach 30 Minuten Rühren bei Raumtemperatur wird erhitzt und 15 Stunden am Rückfluß gekocht. Nach dem Abkühlen der Reaktionsmischung wird das überschüssige $LiAlH_4$ unter Kühlung durch vorsichtige Zugabe von je 10 ml Wasser, 15% NaOH und wieder Wasser zerstört. Das entstandene Aluminiumhydroxid wird abgesaugt und mit Toluol gewaschen. Das Filtrat wird mit der Waschflüssigkeit vereinigt und das Lösungsmittel im Vakuum entfernt. Anschließend wird das Produkt aus Ethanol umkristallisiert. Man erhält 26 g (81%) eines weißen Pulvers vom Schmelzpunkt 71.8 - 72.6 °C.
$^1$H-NMR ($CDCl_3$, 100 MHz)
$\delta$ = 0.9 (t,6H, $-CH_3$); 1.1-2.05 (m,61H, $-CH_2-$,NH); 2.4-2.7 (t, 4H, $N-CH_2$)
20 g (40.5 mmol) N-Hexadecyl-N-octadecylamin, 8.1 g (81 mmol) Bernsteinsäureanhydrid und 8.2 g (81 mmol) Triethylamin werden in 250 ml trockenem Toluol gelöst und die Mischung 2 1/2 Tage unter Feuchtigkeitsausschluß am Rückfluß gekocht. Nach dem Abkühlen wird das Reaktionsgemisch mit 1 M HCl ausgeschüttelt, die organische Phase abgetrennt, mit Na2SO4 getrocknet und das Lösungsmittel im Vakuum entfernt. Anschließend wird zwei Mal aus Methanol umkristallisiert. Man erhält 22.9 g (95 %) eines weißen Pulvers mit Schmelzpunkt 59.8 - 60.7 °C.
$^1$H-NMR ($CDCl_3$, 100 MHz)
$\delta$ = 0.9 (t,6H, $-CH_3$); 1.1-1.9 (m,60H, $-CH_2-$); 2.65 (s,4H, $CO-CH_2-CH_2-CO$); 3.05-3.45 (m,4H, $N-CH_2$)
4.90 g (8.04 mmol) Bernsteinsäure-N-hexadecyl-N-octadecylamid, 1.63 g (12.6 mmol) Methacrylsäure-2-hydroxylester und eine Spatelspitze 2,6-Di-t-butyl-p-kresol werden in 100 ml trockenem Methylenchlorid gelöst und bei 0 °C unter Feuchtigkeitsausschluß eine Lösung von 1.81 g (8.79 mmol) Dicyclohexylcarbodiimid und 35 mg frisch umkristallisiertem N,N-Dimethylaminopyridin in 20 ml trockenem Methylenchlorid zugetropft. Nach beendeter Zugabe wird noch eine Stunde bei 0 °C weiter gerührt. Danach läßt man die Reaktionsmischung bei Raumtemperatur über Nacht stehen und saugt am nächsten Morgen den entstandenen Harnstoff ab. Das Filtrat wird zwei Mal mit 100 ml Wasser gewaschen, mit $Na_2SO_4$ getrocknet und das Lösungsmittel im Vakuum entfernt. Nach säulenchromatographischer Reinigung (Kieselgel Si 60, Laufmittel: Hexan/Ethylacetat 5:1 (v/v)) erhält man 3.93 g (69%) einer weißen, wachsartigen Substanz, die knapp über Raumtemperatur schmilzt.
$^1$H-NMR ($CDCl_3$, 100 MHz)
$\delta$ = 0.9 (t,6H, $-CH_3$); 1.1-1.85 (m,60H, $-CH_2-$); 1.92 (m,3H, $C-CH_3$); 2.65 (t,4H, $CO-CH_2-CH_2-CO$); 3.0-3.45 (m, 4H, $N-CH_2$); 4.34 (s, 4H, $O-CH_2-CH_2-O$); 5.5, 6.1 (AB,2H, $C=CH_2$)

Beispiel 2: Radikalische Polymerisation von Verb. 1

2 g des in Beispiel 1 hergestellten Monomeren werden in 20 ml Tetrahydrofuran gelöst und mit 5.0 mg Azo-bis-isobutyronitril versetzt. Die Lösung wird in einen Dreihalskolben mit Rückflußkühler (mit Gasableitungsrohr und Blasenzähler), Thermometer und Gaseinleitungsrohr gegeben und bei Raumtemperatur eine Stunde lang mit Stickstoff durchspült. Dann wird bis zum Rückfluß erhitzt (Innentemperatur: 65 °C) und acht Stunden unter Rückfluß gekocht. Dabei wird die Reaktionsmischung ständig mittels Magnetrührer gerührt und mit Stickstoff gespült. Das Polymere wird durch Eingießen der Reaktionslösung in 1 l Methanol bei -18 °C ausgefällt und unter Kühlung mit Trockeneis abgesaugt. Man erhält 200 mg einer weißen, bei Raumtemperatur weichen, schmierigen Masse, die in Hexan und Methanol unlöslich und in Tetrahydrofuran löslich ist. Eine Molmassenbestimmung mittels Gelpermeationschromatographie ergab ein Mw von 23000 und ein Mn von 12000 Dalton (Polystyrol-Eichung).

Beispiel 3: Schichtherstellung nach der Langmuir-Blodgett Methode

Ein Glas-Objektträger (76 mm x 26 mm) wird nach folgendem Verfahren gereinigt:
Das Glas wird eine Stunde lang in eine 60 °C warme, frisch angesetzte Mischung aus vier Teilen konz. $H_2SO_4$ und einem Teil 30 %iger $H_2O_2$ gelegt, mit sauberem Wasser abgespült und 15 Minuten lang in einer Reinigungslösung (Extran/ AP 11, Konz. 2-4 g/l) bei 50 °C ultrabeschallt. Danach wird wieder gründlich mit sauberem Wasser abgespült und in einem warmen Luftstrom getrocknet. Anschließend erfolgt zur Hydrophobisierung eine Behandlung mit Hexamethyldisilazan-Dampf (10 Minuten bei 70 °C).

Multischichten aus dem in Beispiel 2 hergestellten Polymeren werden nach dem Verfahren von Langmuir und Blodgett auf den Glasträger übertragen, indem in einer Langmuir-Filmwaage 0.25 $cm^3$ einer Lösung von 5 mg des Polymeren in 10 $cm^3$ einer Mischung aus 1 ml Tetrahydrofuran und 9 ml $CH_2Cl_2$ auf einer wäßrigen Subphase bei einer Subphasentemperatur von 10 °C gespreitet werden. Durch Verkleinerung der monofilmbedeckten Wasseroberfläche wird der Schub auf 25 mN/m eingeregelt und bei diesem Wert konstant gehalten. Der Träger wird nun senkrecht von oben durch die Wasseroberfläche in die Filmwaage eingetaucht (Eintauchgeschwindigkeit: 20 mm/min) und nach einer kurzen Pause (10 sec.) am unteren Umkehrpunkt wieder herausgenommen (Austauchgeschwindigkeit: 10 mm/min) Sowohl beim Eintauch- als auch bei Austauchvorgang wird dabei eine Monolage auf den Träger übertragen. Durch mehrfache Wiederholung des Tauchvorgangs nach jeweils einer Minute Wartezeit am oberen Umkehrpunkt werden insgesamt 20 Doppelschichten übertragen. Die Übertragungsverhältnisse liegen bei 90%. Es werden, auch bei -Übertragung von 50 und mehr Monolagen optisch klare, transparente Schichten erhalten.

Beispiel 4: Ellipsometrische Schichtdicken- und Brechungsindexmessungen

Ein Siliziumplättchen (40 mm x 10 mm) wird aus einem Siliziumwafer herausgeschnitten und wie folgt gereinigt:

1. 1 Stunde Behandlung in einer heißen (60 °C), frisch angesetzten Mischung aus einem Teil 30 %iger $H_2O_2$ und vier Teilen konz. $H_2SO_4$. Anschließend wird mit sauberem Wasser abgespült.
2. 30 Sekunden Eintauchen in $NH_4F$ gepufferte HF-Lösung und anschließend wieder mit sauberem Wasser abspülen.

Nach dieser Behandlung sind die Siliziumplättchen hydrophob (Kontaktwinkel zu Wasser: 75 °).

Schichten eines Polymeren aus Monomer 3 werden wie in Beispiel 3 auf das Siliziumplättchen nach dem Verfahren von Langmuir und Blodgett übertragen (Subphase: Wasser von 10 °C, Schub: 20 mN/m, Eintauchgeschwindigkeit: 20 mm/min, Austauchgeschwindigkeit: 10 mm/min, Pause am oberen Umkehrpunkt: 1 min.). Es werden jeweils sowohl beim Eintauchen als auch beim Austauchen eine Monolage übertragen (Übertragungsverhältnis: 93%). Es werden jeweils Proben mit 10, 30, 50 und 70 Monolagen des Polymeren hergestellt und ellipsometrisch die Schichtdicken und der Brechungsindex der LB-Filme gemessen (Ergebnis: Brechungsindex bei 633 nm: 1.528, Schichtdicke: 1.66 nm/Monolage).

Beispiel 5: Messungen der thermischen Stabilität

Ein Siliziumplättchen (40 mm x 10 mm ) wird aus einem thermisch oxidierten Siliziumwafer (Dicke der Oxidschicht: 160 nm) herausgeschnitten und eine Stunde lang bei 60 °C in eine frisch zubereitete Mischung aus einem Teil 30 %iger $H_2O_2$ und vier Teilen konz. $H_2SO_4$ gelegt. Nach gründlichem Abspülen mit sauberem Wasser wird das Plättchen 15 Minuten lang bei 50 °C in einem Ultraschallbad mit alkalischem Reinigungsbad (Extran$^{(R)}$ AP 11, Konz. 2-4 g/l) behandelt, mit sauberem Wasser gründlich abgespült und in einem warmen Luftstrom getrocknet. Danach erfolgt zur Hydrophobierung eine Behandlung mit Hexamethyldisilazan-Dampf (10 Minuten bei 70 °C).

Die Beschichtung nach der LB-Methode mit erfolgt mit 8 Monolagen eines Polymeren aus Monomer 3 wie in Beispiel 4 beschrieben.

Der beschichtete Träger wird in einer speziellen Apparatur mit linearem Temperaturgradienten (0.5 °C/sec) aufgeheizt. Während des Aufheizvorgangs wird die Dicke der LB-Schicht anhand der Intensität eines von der Probe reflektierten, senkrecht polarisierten Laserstrahl (633 nm) gemessen. Die Temperatur, bei der die erste Änderung der Schichtdicke erfolgt, beträgt bei dem verwendetem Polymeren 110 °C (Zum Vergleich: Bei LB-Schichten aus 22-Tricosensäure beträgt diese Temperatur 70 °C).

Beispiel 6: Messungen der kritischen Oberflächenspannung

Ein Siliziumplättchen (40 mm x 10 mm) wird wie in Beispiel 4 gereinigt und wie in Beispiel 4 mit acht Monolagen des Polymeren aus Monomer 3 beschichtet (Temperatur der Subphase: 10 °C).

Auf die Oberfläche der übertragenen Schichten werden Flüssigkeitstropfen einer Reihe von n-Alkanen ($C_{16}H_{34}$ -$C_{14}H_{30}$) gebracht und die Kontaktwinkel der Flüssigkeitstropfen mit der Oberfläche gemessen. Aus diesen Kontaktwinkeln wird nach dem Verfahren von Zisman die kritische Oberflächenspannung bestimmt. Es ergibt sich ein Wert von 26.5 mN/m. (Zum Vergleich: Bei einer Polyethylen-Oberfläche ergibt sich bei dieser Messung ein Wert von 31 mN/m).

Liste der synthetisierten Monomeren:

| Verb. Nr. | Strukturformel |
|---|---|
| 1 | 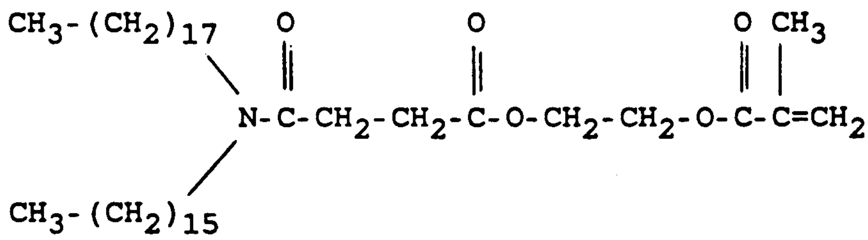 |
| 2 | 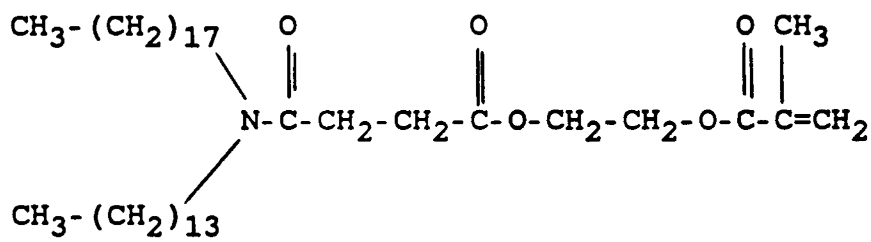 |
| 3 | 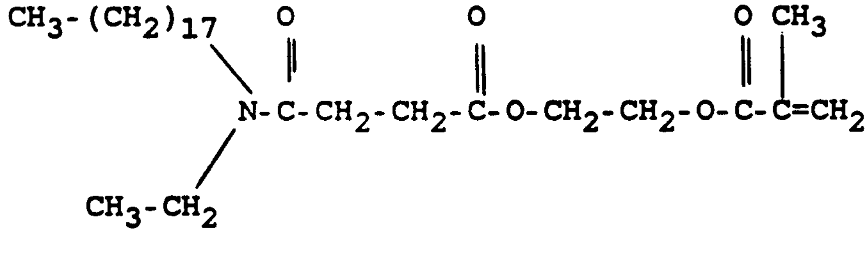 |

## Patentansprüche

**1.** Amphiphile Monomere mit gemischtkettigen Strukturen der Formel

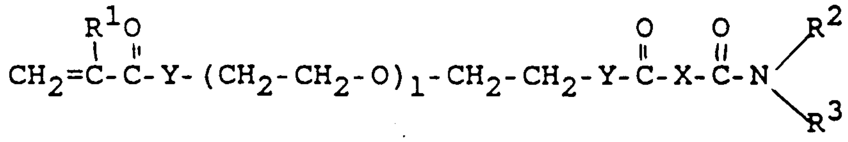

(I)

wobei
Y -O- oder -NH-
X eine Gruppe der Formel $-(CH_2)_n-$ oder $-(CH_2-O-CH_2)_n-$
l eine ganze Zahl von 0 bis 10,
n eine ganze Zahl von 1 bis 10,
$R^1$ Wasserstoff, Methyl, Chlor, Cyan, Fluor oder Brom,
$R^2$ $C_1$-$C_{24}$-Alkyl oder $C_1$-$C_{24}$-Fluoralkyl und
$R^3$ $C_8$-$C_{24}$-Alkyl oder $C_8$-$C_{24}$-Fluoralkyl bedeuten, mit der Maßgabe, daß die Gruppen $R^2$ und $R^3$ eine unterschiedliche Anzahl von C-Atomen enthalten.

**2.** Verfahren zur Herstellung der Monomeren nach Anspruch 1, dadurch gekennzeichnet, daß man eine Verbindung der Formel

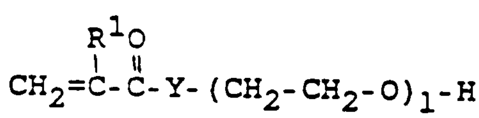

mit einer Verbindung der Formel

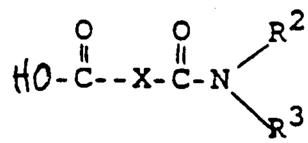

umsetzt, wobei X, Y, l, $R^1$, $R^2$ und $R^3$ die in Anspruch 1 angegebenen Bedeutungen haben, mit der Maßgabe, daß l mindestens 1 ist.

**3.** Polymere erhalten durch Polymerisation eines oder mehrerer Monomere der Formel(I).

**4.** Copolymere erhalten durch Polymerisation eines oder mehrerer Monomere der Formel (I) mit mindestens einem weiteren hydrophilen oder hydrophoben Co-monomeren.

**5.** Copolymere nach Anspruch 4 erhalten durch Polymerisation von 1 bis 20 Gew.-% eines oder mehrerer Monomere der Formel (I) und 99 bis 80 Gew.-% mindestens eines hydrophoben Co-monomers.

**6.** Copolymere nach Anspruch 4 erhalten durch Polymerisation von 10 bis 99 Gew.-% eines oder mehrerer Monomere der Formel (I) und 1 bis 90 Gew.-% mindestens eines hydrophilen Co-monomers.

**7.** Schichtelement mit einem festen Schichtträger und mindestens einer hierauf aufgebrachten festen, dünnen regelmäßig angeordneten Schicht aus einem Polymer nach einem der Ansprüche 3 bis 6.

8. Verfahren zur Herstellung eines Schichtelements gemäß Anspruch7, dadurch gekennzeichnet, daß man mindestens ein Polymer nach einem der Ansprüche 3 bis 6 in einem flüchtigen organischen mit Wasser nicht mischbaren Lösemittel löst, die Lösung an der Grenzfläche Wasser/Luft spreitet, die entstehende Schicht nach Verdunsten des Lösemittels komprimiert und nach der Langmuir-Blodgett-Technik auf einen festen Schichtträger überträgt.

## Claims

1. An amphiphilic monomer having a mixed-chain structure of the formula

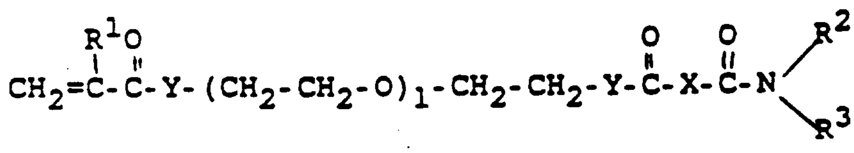 (I)

in which
Y denotes -O- or -NH-
X denotes a group of the formula $-(CH_2)_n-$ or $-(CH_2-O-CH_2)_n-$
l denotes an integer from 0 to 10,
n denotes an integer from 1 to 10,
$R^1$ denotes hydrogen, methyl, chlorine, cyano, fluorine or bromine,
$R^2$ denotes $C_1$-$C_{24}$-alkyl or $C_1$-$C_{24}$-fluoroalkyl and
$R^3$ denotes $C_8$-$C_{24}$-alkyl or $C_8$-$C_{24}$-fluoroalkyl, with the proviso that the groups $R^2$ and $R^3$ contain a different number of carbon atoms.

2. A process for the preparation of the monomer as claimed in claim 1, which comprises reacting a compound of the formula

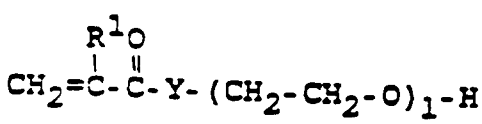

with a compound of the formula

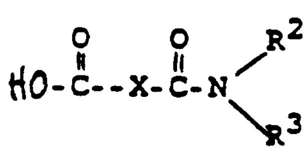

where X, Y, l, $R^1$, $R^2$ and $R^3$ have the meanings given in claim 1, with the proviso that l is at least 1.

3. A polymer obtained by polymerization of one or more monomers of the formula (I).

4. A copolymer obtained by polymerization of one or more monomers of the formula (I) and at least one other hydrophilic or hydrophobic comonomer.

5. A copolymer as claimed in claim 4 obtained by polymerization of l to 20% by weight of one or more monomers of the formula (I) and 99 to 80% by weight of at least one hydrophobic comonomer.

6. A copolymer as claimed in claim 4 obtained by polymerization of 10 to 99% by weight of one or more monomers of the formula (I) and 1 to 90% by weight of at least one hydrophilic comonomer.

**7.** A layer element with a solid layer support and at least one solid, thin, orderly arranged layer of a polymer as claimed in one of claims 3 to 6 applied to the said support.

**8.** A process for the preparation of a layer element as claimed in claim 7, which comprises dissolving at least one polymer as claimed in one of claims 3 to 6 in a volatile organic water-immiscible solvent, spreading the solution on the water/air interface, compressing the resulting layer after evaporation of the solvent and transferring the said layer to a solid layer support by the Langmuir-Blodgett technique.

**Revendications**

**1.** Monomères amphiphiles présentant une structure de chaînes mixtes de formule :

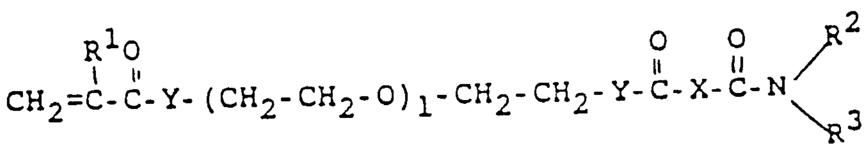

(I)

dans laquelle :
Y représente -O- ou -NH-
X représente un groupe de formule $-(CH_2)_n-$ ou $-(CH_2-O-CH_2)_n-$
l représente un nombre entier compris entre 0 et 10,
n représente un nombre entier compris entre 1 et 10,
$R^1$ représente un atome d'hydrogène, un groupe méthyle, un atome de chlore, un groupe cyano, un atome de fluor ou de brome,
$R^2$ représente un groupe alkyle en $C_1$-$C_{24}$ ou un groupe fluoroalkyle en $C_1$-$C_{24}$ et
$R^3$ représente un groupe alkyle en $C_8$-$C_{24}$ ou un groupe fluoroalkyle en $C_8$-$C_{24}$, sous la condition que les groupes $R^2$ et $R^3$ contiennent un nombre différent d'atomes de carbone.

**2.** Procédé de préparation des monomères selon la revendication 1 caractérisé en ce que l'on fait réagir un composé de formule

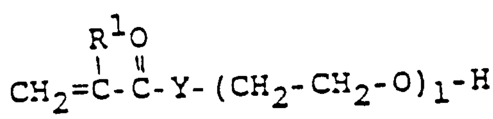

sur un composé de formule

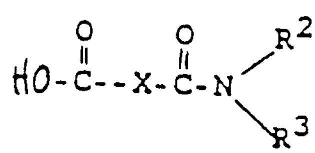

X, Y, l, $R^1$, $R^2$ et $R^3$ présentant dans ces formules les significations indiquées dans la revendication 1 sous la condition que l est au moins égal à 1.

**3.** Polymères obtenus par polymérisation d'un ou de plusieurs monomères de formule (I).

**4.** Copolymères obtenus par polymérisation d'un ou plusieurs monomères de formule (I), avec au moins un comonomère supplémentaire hydrophile ou hydrophobe.

5. Copolymères selon la revendication 4, obtenus par polymérisation de 1 à 20 % en poids d'un ou plusieurs monomères de formule (I) et de 99 à 80 % en poids d'au moins un comonomère hydrophobe.

6. Copolymères selon la revendication 4 obtenus par polymérisation de 10 à 99 % en poids d'un ou plusieurs monomères de formule (I) et de 1 à 90 % en poids d'au moins un comonomère hydrophile.

7. Elément en couches comportant un support de couches solide et au moins une couche déposée sur ce support, cette couche étant solide, mince, régulièrement orientée, et constituée d'un polymère selon l'une des revendications 3 à 6.

8. Procédé de préparation d'un élément en couches selon la revendication 7, caractérisé en ce que l'on dissout au moins un polymère selon l'une des revendications 3 à 6 dans un solvant volatil organique, non miscible à l'eau, on déploie la solution sur l'interface eau/air, on comprime la couche formée après évacuation du solvant, et on dépose ladite couche sur un support de couches solide par la technique de Langmuir-Blodgett.